# EUROPEAN PATENT APPLICATION

(11) **EP 4 744 573 A1**
(43) Date of publication of application: **20.05.2026**
(21) Application number: 24212644.9
(22) Date of filing: 13.11.2024
(51) Int. Cl.: A61B 1/00, A61B 1/05, A61B 1/267

(54) **A VIDEO LARYNGOSCOPE**

(71) Applicant: Kumar, Sujit, Redmond, WA 98052 (US)
(72) Inventor: Kumar, Sujit, Redmond, WA 98052 (US)
(74) Representative: Dr. Gassner & Partner mbB

(57) **Abstract**

The present invention provides a video laryngoscope (100) to perform the intubation process. The video laryngoscope (100) includes a handle (10), a blade (20), a display unit (30), and an attachment assembly (25). The handle (10) includes a camera housing (16) with an image sensor (161) and a light source (162) arranged therein. The image sensor (161) is attached to the handle (10) through a camera channel (15) to visualise the path of the endotracheal tube. The camera housing (16) has a deflector (18) to avoid the blurry and distorted visuals generated due to the scattering of the light from the light source (162). The blade (20) is arranged on the handle (10) to cover the handle (10) and the camera channel (15). Further, the display unit (30) is provided for better visibility of the display screen (34) to visualise the path of the endotracheal tube.

## Description

### TECHNICAL FIELD

The present invention relates to a device for the intubation process. More specifically, the present invention relates to a video laryngoscope.

### BACKGROUND

A laryngoscope is a device, typically comprising a handle and a blade, which is used by clinicians during endotracheal intubation. The laryngoscope assists with intubation by allowing the clinician to visualise the path of the endotracheal tube as the laryngoscope passes through the glottis towards the trachea.

Intubation is the process of inserting a tube, called an endotracheal tube, through the mouth and then into the airway. Intubation is a necessary procedure performed before any surgery that requires a patient to be placed under general anesthesia. Intubation is also performed on patients who need to be placed on a ventilator to assist with breathing. The endotracheal tube is connected to either an anesthesia system or a ventilator, that pushes air into the lungs to deliver a breath to the patient. The laryngoscope is the device used for intubation.

Presently, traditional video laryngoscopes come in two form factors. In the first form factor, the display is detached from the handle and is attached to a cart. The display attached to the cart has a limited manoeuvrability. Also, the size of the display is 7-11 inches and the carts are bulky and hard to move around. While performing the intubation process, the clinician needs to look away from the patient and towards the display which is attached to the cart and not looking at the patient increases the risk of a failed process. In addition, cart-based systems tend to be more expensive and hospitals purchase a few units to be shared between multiple operating rooms. This results in the video laryngoscope not being available for every intubation procedure. In the second form factor, the display is attached to the handle but the displays are small, have poor image quality and are not manoeuvrable. Both form factors result in increased risk to the patient and repeated intubations.

Further, traditional video laryngoscope includes reusable blades that are used again and again for the intubation process. Repeated use of the blades leads to a higher risk of infection, increased operational burden on hospitals and high ownership costs.

Therefore, there is a need for a video laryngoscope, which overcomes few or all drawbacks of the prior art.

### STATEMENT OF THE INVENTION

According to the present invention, there is provided a video laryngoscope. The video laryngoscope may include a handle, a blade, and an attachment mechanism. The handle may have an upper end and a lower end, with a camera channel extending from the lower end. The camera channel may have a camera housing with an image sensor to capture and transmit visuals of the patient's airway during intubation. The blade may be slidably attachable to the handle to cover the entire surface of the handle in an attached position. The blade may be securely attached using the attachment mechanism, which may be arranged at the upper end of the handle.

The attachment mechanism may include at least two ball projections that are biased to extend radially outward from the center of the handle. The two ball projections may engage corresponding openings in the blade to securely hold the blade in place during the intubation process. The two ball projections may be biased using a biasing member, which pushes the two ball projections outward from the center of the handle. The blade may be detached from the handle by pressing the ball projections inward, overcoming the biasing force, and disengaging the two ball projections from the corresponding openings in the blade.

The blade may include an attaching portion, a gripping portion, and a tongue portion. The attaching portion may be adapted to connect with the handle using the attachment mechanism. The gripping portion may include surface indentations to provide grip for the clinician during the intubation procedure. The tongue portion may accommodate the camera channel and include a camera housing opening configured to receive a camera housing, enabling the camera to capture visuals of the airway, larynx, trachea, and endotracheal tube. The tongue portion may be adapted to facilitate smooth insertion into the patient's airway.

The handle may further include a tapered portion at the lower end, from which the camera channel extends. The camera channel may include a camera housing at its distal end to capture and transmit visuals of the airway, trachea, or endotracheal tube to a connected display unit.

Additionally, the handle may include a receiving port adapted to receive a display unit. The receiving port may house an attachment assembly, allowing the display unit to be securely attached to and detached from the handle. The handle may include terminals exposed within the receiving port that allow for an electrical connection between the camera channel and the display unit, enabling real-time visual feedback during intubation.

In another aspect, the handle may include a controller arranged within the body of the handle. The controller may connect the camera channel to the display unit, allowing the captured visuals to be transmitted to the display unit. The terminals in the receiving port may include a first terminal in the handle and a second terminal on the display unit, which can establish an electrical connection between the camera and the display unit, ensuring that visuals of the airway are properly displayed during the intubation procedure.

The blade may be designed to enclose the handle and the camera channel within cavities formed in the attaching portion and the gripping portions. These cavities may allow the blade to slide over the handle and fully enclose it in an attached position. The tongue portion of the blade may include a camera housing opening near a tip, through which the camera can capture visuals of the patient's airway to enable the clinician to guide the endotracheal tube into the trachea with real-time visual feedback.

In the second aspect, a handle for a video laryngoscope is provided, which may include a body with an upper and lower end, and a controller. The upper end of the handle may include a receiving port adapted to receive a display unit and may facilitate the attachment of the blade to the handle. The lower end may be configured to connect the camera channel to the handle. The controller arranged within the handle may connect the camera channel to the display unit, enabling the transmission of visuals from the image sensor to the display unit. The terminals within the receiving port and the display unit may allow for the establishment of an electrical connection, facilitating the display of captured visuals.

In another aspect, the blade may be configured for use with the video laryngoscope, preferably with a handle and may include an attaching portion, a gripping portion, and a tongue portion. The blade may be adapted to slide over the handle and the camera channel, securely accommodating the handle within cavities formed in the attaching portion and the gripping portions. The blade may include at least two openings that engage with the two ball projections of the handle, securing the blade in place during intubation. The tongue portion of the blade may have a camera housing opening near the tip, allowing the camera housing to capture visuals of the airway, larynx, or trachea.

Further, the blade may include a first cavity in the attaching portion and the gripping portion to accommodate the handle, and a second cavity in the tongue portion to accommodate the camera channel. The first cavity and the second cavity accommodate both the handle and the camera channel during the intubation process. Additionally, the blade may include surface indentations on the gripping portion to provide a e grip for the clinician during the intubation process.

### BRIEF DESCRIPTION OF DRAWINGS

Other features and advantages of the invention will become apparent when reading the detailed description given below, purely by way of example and in a non-limitative manner, referring to the following figures:
FIG. 1 illustrates a side view of a video laryngoscope in accordance with the present invention;
FIG. 2 illustrate a front view of a handle of the video laryngoscope in accordance with the present invention;
FIG. 3 illustrate a side view of a handle of the video laryngoscope in accordance with the present invention;
FIG. 4a-4c illustrate a front view of the handle while detaching a blade in accordance with the present invention;
FIG. 5 illustrate a side view of the blade of the video laryngoscope in accordance with the present invention;
FIG. 6 illustrate a side view of the video laryngoscope in accordance with the second embodiment;
FIG. 7a-7b illustrate a front view and a side view of a handle of the video laryngoscope in accordance with the embodiment shown in FIG. 6;
FIG. 8 illustrates a perspective view of a camera housing the video laryngoscope in accordance with the present invention;
FIG. 9 illustrates a perspective view of a deflector of the video laryngoscope in accordance with the present invention;
FIG. 10 illustrates a front view of a display assembly of the video laryngoscope in accordance with the present invention; and
FIG. 11 illustrates a bottom view of a display cover of the video laryngoscope in accordance with the present invention.

### DETAILED DESCRIPTION

An embodiment of this invention, illustrating its features, will now be described in detail. The words "comprising, "having, "containing," and "including," and other forms thereof, are intended to be equivalent in meaning and be open ended in that an item or items following any one of these words is not meant to be an exhaustive listing of such item or items, or meant to be limited to only the listed item or items.

The present invention provides a video laryngoscope. The video laryngoscope is adapted to seamlessly attach and detach a display unit and a blade from a handle. The video laryngoscope is adapted to avoid the blurry and distorted visuals of the path of the endotracheal tube. Further, the video laryngoscope is adapted to control the infection rate which is spread due to the regular laryngoscopes. Furthermore, the video laryngoscope allows the clinicians to perform the intubation process without sacrificing the visibility of the display unit.

The terms "first," "second," and the like, herein do not denote any order, quantity, or importance, but rather are used to distinguish one element from another, and the terms "an" and "a" herein do not denote a limitation of quantity, but rather denote the presence of at least one of the referenced item. The disclosed embodiments are merely exemplary of the invention, which may be embodied in various forms.

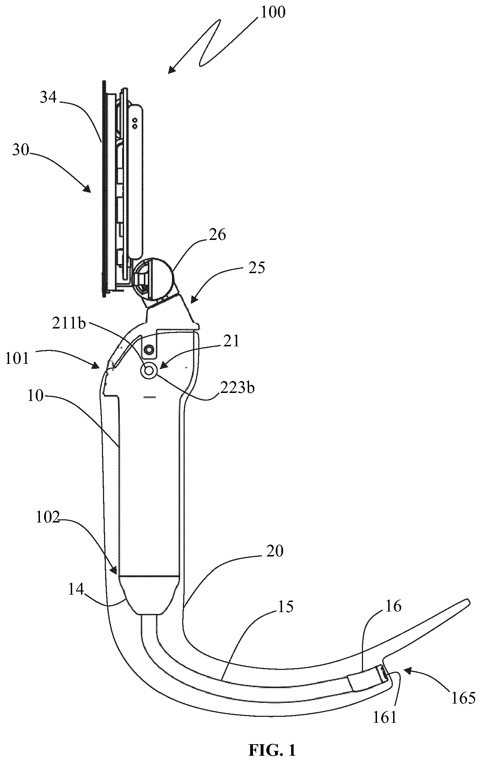

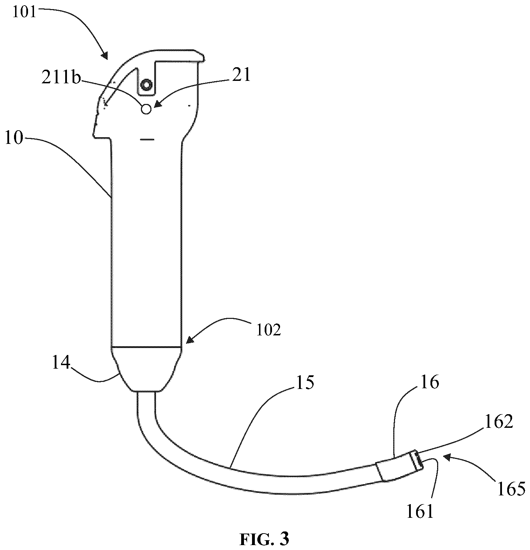

Referring to FIG. 1, 2, and 3, a video laryngoscope (100) in accordance with the present invention is illustrated. The video laryngoscope (100) includes a handle (10), a blade (20), and an attachment mechanism (21) to attach the blade (20) to the handle (10). The handle (10) is an elongated member that has an ergonomic design that allows clinicians to easily hold the handle (10) during an intubation process. The handle (10) has an upper end (101) and a lower end (102). The upper end (101) of the handle (10) is provided with a receiving port (12) to receive a display unit (30).

The lower end (102) of the handle (10) is provided with a tapered portion (14). Specifically, the lower end (102) of the handle (10) is provided with a camera channel (15) that extends from the tapered portion (14) of the handle (10). The camera channel (15) is a flexible link extending from the tapered portion (14) of the handle (10) and comprises an electric circuit to transfer the visuals like images and videos to the receiving port (12). The camera channel (15) has a first end connected to the tapered portion (14) of the handle (10) and a second end that is a distal end of the camera channel comprising a camera housing (16). The camera housing (16) of the camera channel (15) is provided to capture visuals of the path of the endotracheal tube, larynx, trachea or airway.

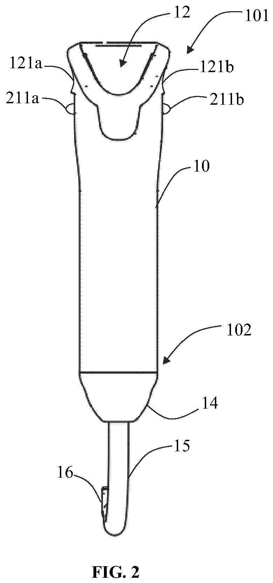

Referring to FIG. 1, and 2, the handle (10) is adapted to receive the blade (20) from the lower end (102) of the handle (10). The blade (20) is received around the outer surface of the handle (10) to entirely cover the handle (10) and the camera channel (15). The blade (20) is attached to the handle (10) while performing the intubation process. The handle (10) is adapted to receive all types of blades like Macintosh, and Miller or both adult blades and child blades. Further, the camera channel (15) is adapted to receive all types of blades like Macintosh, and Miller for both adults and children. The handle (10) does not require any other adjustments or mountings to receive adult blades and child blades. The blade (20) is slidable over the camera channel (15) to attach with the handle (10) to perform the intubation process. The blade (20) is detachably attached to the handle (10) and is detachable from the handle (10) after the intubation process.

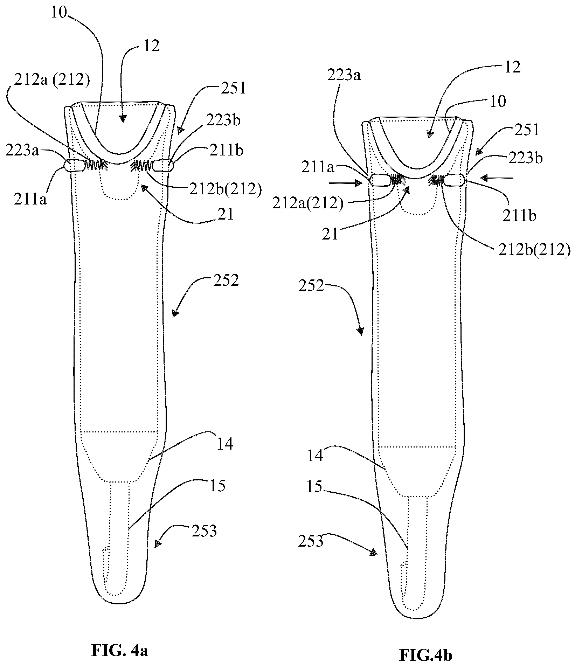

Referring now to FIG. 4a-4c, in the present embodiment of the invention, the blade (20) is attachable to the handle (10) through an attachment mechanism (21) of the handle (10). The attachment mechanism (21) includes at least two ball projections (211a, 211b) arranged opposite to each other to securely attach and detach the blade (20) to the handle (10). The two ball projections (211a, 211b) are biased to radially extend away from the centre axis of the handle (10). The two ball projections (211a, 211b) are configured to engage with the blade (20). Specifically, the two ball projections (211a, 211b) are biased to extend radially outward from the center of the handle (10) using a biasing member (212) such as springs. The biasing member (212) is positioned inside the handle (10) and applies a continuous force to the ball projections (211a, 211b), to configure an extended position of two ball projections (211a, 211b) during the attachment of the blade (20). The two ball projections (211a, 211b) have corresponding biasing members (212a, 212b) to bias the two ball projections (211a, 211b) away from the handle (10) to engage and hold the blade (20) in an attached position. When the blade (20) is attached, the ball projections (211a, 211b) engage with corresponding openings (223a, 223b) in the blade (20), holding the blade (20) around the handle (10).

In the present embodiment, the biasing member (212) is a compression spring. It may be obvious for a person skilled in the art to use any other type of spring as a biasing member (212).

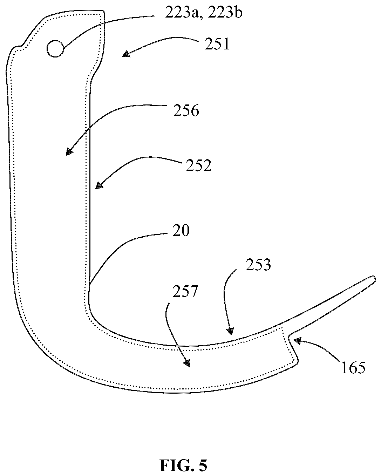

Referring now to FIG. 5, the blade (20) includes an attaching portion (251), a gripping portion (252), and a tongue portion (253). The attaching portion (251) is at the proximal end of the blade (20) and is adapted to attach the blade (20) to the handle (10). The attaching portion (251) includes at least two openings (223a, 223b) positioned to engage with the two ball projections (211a, 211b) of the handle (10). The two openings (223a, 223b) are provided to engage with the corresponding two ball projections (211a, 211b) during the intubation process. The attachment mechanism (21) allows the detachment of the handle (10) by pressing the two ball projections (211a, 211b) inward. The blade (20) has a first cavity (256) formed at the attaching portion (251) and the gripping portion (252) of the blade (20). The first cavity (256) is having a shape that is adapted to fit over the handle (10) and cover the handle (10) during the intubation process. The first cavity (256) allows the blade (20) to accommodate the handle (10) and remain firm in place during the intubation process. The first cavity (256) is formed at the attaching portion (251) and extends through the gripping portion (252).

The gripping portion (252) is a middle section of the blade and is arranged between the attaching portion (251) and the tongue portion (253). The gripping portion (252) is configured to provide a grip for the clinician during the intubation process. The gripping portion (252) has indentations (not shown) or other surface textures on the outer surface of the gripping portion (252) to enhance the grip of the clinician.

Furthermore, the tongue portion (253) is a distal end of the blade (20) located after the gripping portion (252). The tongue portion (253) is provided to interact with the patient's anatomy, particularly during intubation. In the present embodiment, the tongue portion (253) is an integral part of the blade (20) and transversally extends from the gripping portion (252). The tongue portion (253) is configured to receive the camera channel (15) and the camera housing (16). The tongue portion (253) includes a camera housing opening (165) to accommodate the camera housing (16) facing outside of the blade (20) allowing an image sensor (161) arranged within the camera housing (16) to capture visuals of the patient's airway, including the endotracheal tube, larynx, and trachea.

Specifically, the blade (20) has a second cavity (257) formed at the tongue portion (253) of the blade (20). The second cavity (257) is configured to accommodate the camera channel (15) and the camera housing (16) therein. The second cavity (257) accommodates the camera channel (15), which runs along the length of the tongue portion (253). The second cavity (257) provides space for the camera channel (15) to be housed within the blade (20). Further, the camera housing (16) is exposed through the camera housing opening (165) arranged in the tongue portion (253) to allow the image sensor (161) to capture the visuals of the patient's airway.

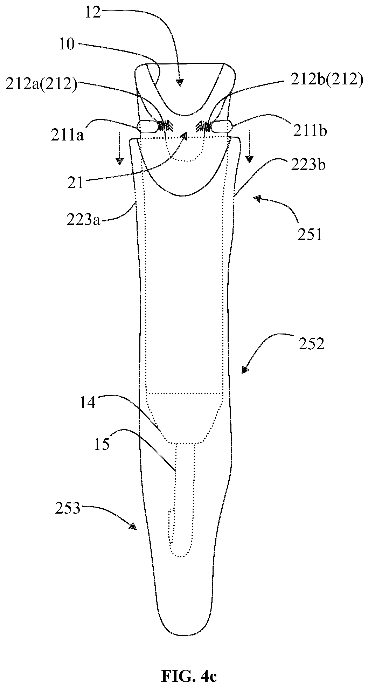

After the intubation process, the blade (20) is detachable from the handle (10) by pressing the two ball projections (211a, 211b) against the biasing force exerted by the biasing members (212a, 212b). When the pressure to the two ball projections (211a, 211b) is applied, the two ball projections (211a, 211b) are pushed inward, overcoming the biasing force of the biasing members (212a, 212b) and moving toward the center of the handle (10) (as shown in FIG. 4b). The two ball projections (211a, 211b) are moved towards the center of the handle (10) to disengage from the corresponding openings (211a, 211b) in the blade (20), allowing the blade (20) to be detached from the handle (10). Particularly, upon pressing the two ball projections (211a, 211b), the blade (20) is slidable over the handle (10) and the camera channel (15) in a downward direction (shown in FIG. 4c), thereby completely removing the blade (20) from the handle (10) and disposing of it after the intubation process.

The blade (20) is disposed of after detaching from the handle (10) to reduce the chance of infection and cross-contamination. Disposing of the blade (20) after one use allows the video laryngoscope (100) to control the spread of infection which can be spread by using the same blade again and again.

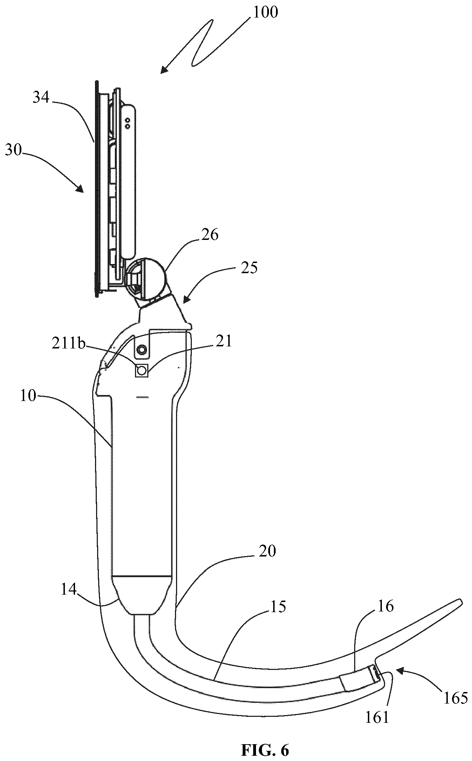

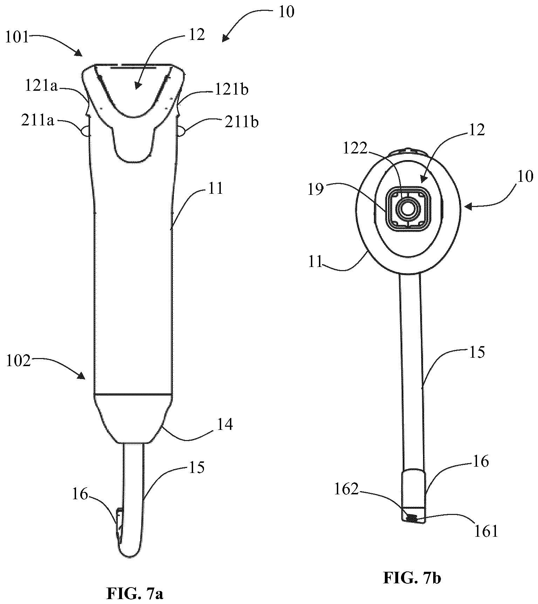

Referring now to FIG. 6, and 7a-7b, a handle (10) for a video laryngoscope (100) in accordance with a second embodiment of the present invention is illustrated. The handle (10) includes a body (11) and a controller (19) arranged within the body (11). The body (11) has an upper end (101) and a lower end (102). The upper end (101) is adapted to receive a display unit (not shown) through a receiving port (12) and connect the blade (20) to the handle (10) simultaneously. The upper end (101) of the handle (10) has an attachment mechanism (21) arranged within the receiving port (12) to attach the blade (20) to the handle (10). The attachment mechanism (21) includes at least two ball projections (211a, 211b) extending radially away from the center of the handle (10). The two ball projections (211a, 211b) are engageable with the blade (20) to securely hold the blade (20) in an attached position.

The lower end (102) is configured to connect a camera channel (15) to the handle (10). Specifically, the handle (10) has a tapered portion (14) arranged at the lower end (102) of the body (11) to guide the blade (20) while sliding over the handle (10) for attaching the blade (20) to the handle (10). Further, the camera channel (15) extends from the tapered portion (14) and is connected to the controller (19).

The controller (19) is arranged within the body (11). The controller (19) is adapted to electrically connect with the display unit (30) and the camera channel (15). Specifically, the controller (19) is adapted to connect with the display unit (30) through terminals exposed within the receiving port (12) when the display unit (30) is attached to the handle (10). The terminals are provided to establish a connection between the display unit (30) and the camera channel (15) to send visuals of the path of the endotracheal tube, larynx, trachea, or airway to the display unit (30).

Specifically, the handle (10) includes a first terminal (122) arranged within the receiving port (12) at the upper end (101) of the handle (10). The first terminal (122) is configured to connect electrically to a corresponding second terminal (28) located on the display unit (30). The first terminal (122) and the second terminal (28) are configured to establish an electrical connection between the handle (10) and the display unit (30) when the display unit (30) is attached.

The first terminal (122) provides a connection interface for the image sensor (161) housed within the camera housing (16). When the display unit (30) is attached to the receiving port (12), the second terminal (28) on the display unit (30) aligns with the first terminal (122) on the handle (10), establishing an electrical connection. The electrical connection enables the transmission of visuals captured by the image sensor (161), which is located in the camera housing (16) at the distal end of the camera channel (15).

When the display unit (30) is attached to the handle (10), the controller (19) receives the electrical signal from the image sensor (161) and transmits the electrical signal to the display unit (30). The controller (19) processes and displays the visuals of the trachea, larynx, or airway on the screen (34) of the display unit (30), allowing the clinician to monitor the intubation process in real-time. The electrical connection between the image sensor (161), the controller (19), and the display unit (30) facilitates visual feedback throughout the intubation process. The first terminal (122) and the second terminal (28) allow the display unit (30) to be quickly attached or detached from the handle (10) without any need for additional wiring.

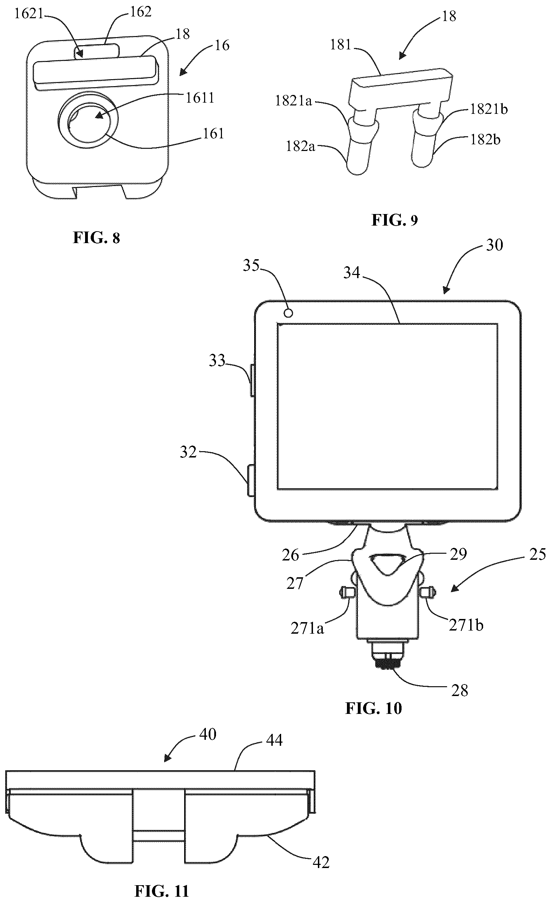

Referring now to FIG. 8, the camera housing (16) comprises an image sensor (161) and a light source (162). The image sensor (161) facilitates capturing of the visuals of the path of the endotracheal tube, larynx, trachea or airway. Specifically, an image sensor opening (1611) in the camera housing (16) allows the image sensor (161) to capture the visuals of the path of the endotracheal tube, larynx, trachea or airway. In the present aspect of the invention, the image sensor (161) is a 2-megapixel (MP) camera, but a person skilled in the art can use any other image sensor (161) having different specifications.

Further, the camera housing (16) includes a light source opening (1621) to allow the light source (162) to illuminate the path of the endotracheal tube and the airway. Furthermore, the camera housing (16) has a deflector (18). Specifically, the deflector (18) is arranged on an outer surface of the camera housing (16) separating the image sensor opening (1611) and the light source opening (1621). The deflector (18) avoids the direct exposure of the light source (162) to the image sensor (161).

In the present aspect of the invention, the deflector (18) is a rectangular strip. The rectangular strip is made up of a silicone material. A person skilled in the art can configure the deflector (18) with any other material, shape or size according to the size and position of the image sensor (161) and the light source (162). The deflector (18) is horizontally arranged between the light source opening (1621) and the image sensor opening (1611) to avoid the scattering of the light on the image sensor (161).

Referring now to FIG. 8, and 9, the deflector (18) includes a rectangular portion (181), and two legs (182a, 182b) extending from the ends of the rectangular portion (181). The two legs (182a, 182b) are provided with respective locking elements (1821a, 1821b) to attach the deflector (18) to the camera housing (16). Specifically, the camera housing (16) has two openings (not shown) to receive the locking elements (1821a, 1821b) of the two legs (182a, 182b) to hold the deflector (18) on the outer surface of the camera housing (16). The deflector (18) is fixedly attached to the camera housing (16) using the locking elements (1821a, 1821b) but a person skilled in the art can provide the deflector (18) which is detachable from the camera housing (16). Specifically, the deflector (18) is detachable from the camera housing (16) if the deflector (18) has incurred damage or requires replacement.

In another aspect of the invention, the deflector (18) is an extruded portion (not shown) that extends from the outer surface of the camera housing (16). The extruded portion is provided between the image sensor opening (1611) and the light source opening (1621) to avoid direct light exposure to the image sensor (161) from the light source (162). The scattering of the light from the light source (162) makes images distorted and blurry. The deflector (18) avoids direct exposure of the light source (162) to the image sensor (161) to avoid distortion and blurriness of the images.

In another aspect of the invention, the deflector (18) is a thin sheet arranged between the image sensor opening (1611) and the light source opening (1621). The sheet can be made up of any material that absorbs light to avoid the direct exposure of the light source (162) on the image sensor (161).

Referring now to FIG. 1, and 10, the display unit (30) is provided which is attachable to the handle (10) through the attachment assembly (25). The attachment assembly (25) includes a housing (27), and the second terminal (28) which is electrically connected to the display unit (30). The housing (27) is connected to the display unit (30) using a hinge (26). The hinge (26) is provided to pivot the display unit (30) in a back-and-forth direction. The hinge (26) is connected to the display unit (30) and the housing (27). Specifically, the hinge (26) has a connecting element (not shown) that is adapted to attach to the housing (27). The housing (27) receives the connecting element of the hinge (26) to allow the display unit (30) to freely rotate around the central axis of the handle (10). The hinge (26) allows the display unit (30) to rotate in all the possible directions to configure the display unit (30) in a required position to provide better visibility to the clinician.

Further, the housing (27) includes at least two ball projections (271a, 271b) extending from an outer surface of the housing (27) and arranged opposite to each other to securely attach and detach the housing (27) to the handle (10). The two ball projections (271a, 271b) are biased to radially extend away from the centre axis of the housing (27). The housing (271a, 271b) is attached to the receiving port (12) (shown in FIG. 7a) of the handle (10). Specifically, the receiving port (12) has two openings (121a, 121b) to receive the respective ball projections (271a, 271b) of the housing (27) to securely hold the housing (27) within the receiving port (12) of the handle (10). The two ball projections (271a, 271b) ensure that the housing (27) is held in place until the two ball projections (271a, 271b) are pressed to detach the housing (27) of the attachment assembly (25). Specifically, the two ball projections (271a, 271b) have respective balls arranged at the extreme ends to easily press the two ball projections (271a, 271b) while detaching the housing (27) from the handle (10).

Once the display unit (30) is attached to the handle (10) through the attachment assembly (25), the second terminal (28) of the attachment assembly (25) electrically connects with the first terminal (122) of the receiving port (12) of the handle (10) to establish the electric connection between the camera channel (15) and the display unit (30). Specifically, upon attaching the attachment assembly (25) to the handle (10), the first terminal (122) and the second terminal (28) immediately establish an electric connection between the image sensor (161) and the display unit (30) enabling the instantaneous display of visuals of the path of the endotracheal tube, larynx, trachea, or airway on the display screen (34).

Furthermore, the attachment assembly (25) includes a record button (29) (as shown in FIG. 10) to capture an image or record a video. In the present aspect of the invention, the record button (29) is arranged on a portion of the housing (27). However, a person skilled in the art can arrange the record button (29) on any other portion in such a way that the clinician's thumb easily reaches to the record button (29) to capture an image or record a video.

Referring now to FIG. 10, the display unit (30) includes a battery (not shown), a power button (32), an output port (33), a display screen (34), a touch user interface (TUI) (not shown), a processor (not shown), and a memory (not shown) to store the visuals of the path of the endotracheal tube, larynx, trachea or airway. The battery is arranged within the display unit (30) to supply power to the display screen (34), the image sensor (161) and the light source (162). The battery can be a replaceable battery or a rechargeable battery.

In another aspect of the invention, the handle (10) includes a battery (not shown) that can be used to supply power to the image sensor (161) and the light sensor (162) when the display unit (30) is not attached to the handle (10). In such cases, the handle (10) establishes a wireless connection with the display unit (30) or an external display (not shown) to transfer the visuals of the path of the endotracheal tube, larynx, trachea or airway from the image sensor (161) to the display unit (30) or the external display.

The power button (32) is provided on any of the sides of the display unit (30). The power button (32) is within the range of the clinician's fingers. In the present aspect of the invention, the power button (32) is arranged at the bottom left of the display unit (30). The power button (32) is used to power on or power off the display screen (34). Specifically, the power button (32) is pressed and released to power on the display screen (34), and the power button (32) is pressed and held for 3-4 seconds to power off the display screen (34). A person skilled in the art can configure the duration for holding the power button (32) to power off the display screen (34).

In another aspect of the invention, the display screen (34) starts displaying the visuals of the path of the endotracheal tube once the display unit (30) is attached to the handle (10) and the display screen (34) is powered ON using the power button (32). The display screen (34) automatically starts displaying the visual of the endotracheal tube without pressing the record button (29).

In one more aspect of the invention, the video laryngoscope (100) is adapted to start displaying the visuals on the display screen (34) even if the display unit (30) is not attached to the handle (10). The display screen (34) displays the visuals by establishing the wireless connection between the handle (10) and the display unit (30).

Further, the output port (33) is provided on any of the sides of the display unit (30) to connect the display unit (30) to an external display (not shown) to show the visuals of the path of the endotracheal tube. The external display has a bigger screen size than the display screen (34) of the display unit (30). It is obvious to a person skilled in the art to provide a plurality of output ports on the display unit (30).

The display screen (34) of the display unit (30) is adapted to show visuals of the path of the endotracheal tube received from the image sensor (161). In the present aspect of the invention, the display unit (30) has a rectangular shape to accommodate the display screen (34). The display screen (34) has a size of 3.5 inches to provide better visibility from all angles. It is obvious to a person skilled in the art to increase the size of the display screen (34) according to the requirement.

Further, the touch user interface is provided to allow the clinician to interact with the video laryngoscope (100). The touch user interface of the display screen (34) is adapted to review and manage previously recorded images or videos which are stored in the memory of the display unit (30). The touch user interface includes a home page to show the current time and date, a menu icon to access the plurality of icons, and a battery icon to indicate the level of the battery. The display screen (34) is powered on using the power button (32) to display the home page. The clinician interacts with the menu icon of the home page by touching the menu icon displayed on the display screen (34) to access the plurality of the icons. The menu icon is provided on the bottom left corner of the display screen (34) to allow the clinician to reach the menu icon with one hand. After touching the menu icon, the processor receives the touch input from the touch user interface and processes the command to show the plurality of icons on the display screen (34).

The plurality of icons is assigned with specific actions to perform after receiving the command from the processor of the display unit (30). Specifically, the plurality of icons includes a settings icon to make changes in the brightness of the display screen, the volume of the sound, turn on or turn off the output port (33), and the like. Further, the plurality of icons includes a photo icon to view and manage previously recorded images, and a video icon to view and manage previously recorded videos. In another aspect of the invention, the plurality of icons includes a folder icon to view and manage the previously recorded images and videos in one place. A person skilled in the art can add more icons to the plurality of the icons to provide additional features and functions.

The touch user interface is adapted to show an indication of the captured image on the display screen (34) when the record button (29) is pressed by the clinician to capture the image. Further, the touch user interface is adapted to show a timer of the recording on the display screen (34) when the record button (29) is pressed and held to record the video. Further, the timer disappears from the display screen (34) after pressing the record button (29) to stop the recording.

In another aspect of the present invention, the display unit (30) includes a charging light (35) to indicate the charging status of the battery. The charging light (35) turns on when the battery is connected to a power source to recharge the battery and turns off when the battery is not connected to the power source for recharging. It is obvious to a person skilled in the art to arrange the charging light (35) in any of the corners of the display unit (30).

Referring now to FIG. 11, a display cover (40) is provided to prevent the display unit (30) and the attachment assembly (25) (shown in FIG. 10) from getting infected during the intubation process. The display cover (40) is made up of a transparent material to allow the clinician to view the display screen (34) without any hindrance. The display cover (40) includes a lower panel (42) and an upper panel (44) hinged at a hinge point. The lower panel (42) and the upper panel (44) pivot around the hinge point to cover the display unit (30). The upper panel (44) and the lower panel (42) of the display cover (40) are pressed to snap fit with each other after placing the display unit (30) within a cavity of the display cover (40). Further, the upper panel (44) has a flexible sheath (not shown) extending from the free end of the upper panel (44) to cover the attachment assembly (25) and a portion of the handle (10). The display cover (40) is disposable and can be disposed of once the intubation process is finished.

Before performing the intubation process, the clinicians attach the blade (20) to the handle (10). The blade (20) can be an adult blade or a child blade. The blade (20) is slidable over the camera channel (15) and is attached to the handle (10) through the dual ball mechanism (21) of the handle (10). Further, the display unit (30) is placed inside the lower panel (42) of the display cover (40) and the upper panel (44) is pivoted to close the display cover (40). The flexible sheath is wrapped around the attachment assembly (25) and the portion of the handle (10). Once the flexible sheath is wrapped, the video laryngoscope (100) is ready to use for the intubation process. The display unit (30) is rotated to configure the position according to the clinician's requirement. The movement of the display unit (30) is facilitated by the hinge (26).

While performing the intubation process, the power button (32) is pressed to activate the display screen (34), the image sensor (161), and the light source (162). The visuals of the path of the endotracheal tube and the airway are displayed on the display screen (34). The deflector (18) of the camera housing (16) reduces the scattering of the light from the light source (162). The deflector (18) helps to reduce the distortion and blurriness of the images. Further, the record button (29) is provided to capture the image and record the video. The record button (29) is pressed and released to capture the image, and the record button (29) is pressed and held for 2 to 3 seconds to record the video. The video laryngoscope (100) is completely covered from the outside eliminating the risk of the video laryngoscope (100) getting infected. After the intubation process, the display cover (40) is removed from the display unit (30) and disposed of to avoid spreading infection. Further, the blade (20) is removed from the handle (10). The blade (20) which is mainly in contact with the body parts is disposed of after one use which helps to reduce the rate of infection or cross-contamination spread due to the repetitive use of the blade (20).

Further, the video laryngoscope (100) allows the clinician to have one hand free while operating the video laryngoscope (100) during the intubation process. In an operating room, clinicians need free hands to simultaneously use multiple devices or perform multiple actions. With the placement of the power button (32) at the bottom left of the display unit (30), the menu icon on the bottom left of the display screen (34) and the record button (29) under the display unit (30), the clinician can fully operate the video laryngoscope (100) with their left hand thus leaving the right hand free.

In another aspect of the present invention, the video laryngoscope (100) includes a handle (10), a blade (20), and a display unit (30). The display unit (30) is attached to the handle (10) without using an attachment assembly (25). Specifically, the display unit (30) includes an attaching element (not shown) extending from a bottom portion of the display unit (30) to attach with the handle (10). It is obvious to a person skilled in the art to configure the attaching element of the display unit (30) to provide a threaded attachment or a snap-fit attachment or a similar attachment, to ensure a secure and functional connection between the display unit (30) and the handle (10).

Therefore, the present invention has the advantage of providing a video laryngoscope (100) to perform the intubation process. Further, the handle (10) of the video laryngoscope (100) is adapted to receive the adult blades as well as child blades, which reduces the other requirements like different handles for different blades or mountings to receive the different types of blades on the same handle (10). Further, the deflector (18) reduces the distortion and blurriness of the images for better quality visualisation. Furthermore, the handle (10) is covered by the blade (20) and the display unit (30) is covered by the display cover (40) which completely covers the video laryngoscope (100) and prevents the video laryngoscope (100) from getting infected during the intubation process.

The foregoing descriptions of specific embodiments of the present invention have been presented for purposes of illustration and description. They are not intended to be exhaustive or to limit the present invention to the precise forms disclosed, and obviously, many modifications and variations are possible in light of the above teaching. The embodiments were chosen and described in order to explain the principles of the present invention best and its practical application, to thereby enable others skilled in the art to best utilise the present invention and various embodiments with various modifications as are suited to the particular use contemplated. It is understood that various omission and substitutions of equivalents are contemplated as circumstance may suggest or render expedient, but such are intended to cover the application or implementation without departing from the scope of the claims of the present invention.

## Claims

1. A video laryngoscope (100) comprising:
a handle (10) having an upper end (101) and a lower end (102), the handle (10) has a camera channel (15) extending from the lower end (102) of the handle (10);
a blade (20) attachable to the handle (10), and slidable over the camera channel (15) and the handle (10) to cover an entire surface of the handle (10) in an attached position; and
an attachment mechanism (21) to attach the blade (20) to the handle (10), the attachment mechanism (21) is arranged within the handle (10) at the upper end (101) of the handle (10),
wherein, the attachment mechanism (21) includes at least two ball projections (211a, 211b), biased to radially extend away from a centre of the handle (10), the two ball projections (211a, 211b) are adapted to engage with corresponding openings (223a, 223b) of the blade (20) to hold the blade (20) around the handle (10) during the intubation process.

2. The video laryngoscope (100) as claimed in claim 1, wherein the two ball projections (211a, 211b) are biased away from the centre of the handle (10) using a biasing member (212), the two ball projections (211a, 211b) have corresponding biasing members (212a, 212b) to bias the two ball projections (211a, 211b) away from the handle (10) to engage and hold the blade (20) in the attached position.

3. The video laryngoscope (100) as claimed in claim 1, wherein the blade (20) is detachable from the handle (10) by pressing the two ball projections (211a, 211b) against a biasing force, upon pressing, the two ball projections (211a, 211b) overcome the biasing force of the biasing member (212) to move towards the centre of the handle (10) thereby disengaging the two ball projections (211a, 211b) from the corresponding openings (223a, 223b) of the blade (20) to detach the blade (20) from the handle (10).

4. The video laryngoscope (100) as claimed in claim 1, wherein the blade (20) includes an attaching portion (251), a gripping portion (252), and a tongue portion (253), the attaching portion (251) is adapted to connect with the handle (10) through the attachment assembly (25), wherein the attaching portion (251) has at least two openings (223a, 223b) arranged opposite to each other and are engageable with the two ball projections (211a, 211b) of the handle (10), wherein the gripping portion (252) has indentations on an outer surface to provide grip to a clinician or a healthcare physician, during the intubation process, wherein the tongue portion (253) is adapted to receive the camera channel (15) and a camera housing (16) arranged at the end of the camera channel (15), wherein the tongue portion (253) has a camera housing opening (165) configured to receive the camera housing (16) facing outside of the blade (20) to capture visuals of an endotracheal tube, larynx, trachea, or airway.

5. The video laryngoscope (100) as claimed in claim 4, wherein the blade (20) has a first cavity (256) formed at the attaching portion (251) and the gripping portion (252), and a second cavity (257) formed at the tongue portion (253) to accommodate the handle (10) and a camera channel (15) respectively.

6. The video laryngoscope (100) as claimed in claim 4, wherein the tongue portion (253) is adapted to receive the camera channel (15) and a camera housing (16) arranged at the end of the camera channel (15), wherein the tongue portion (253) has a camera housing opening (165) configured to receive the camera housing (16) facing outside of the blade (20) to capture visuals of an endotracheal tube, larynx, trachea, or airway.

7. The video laryngoscope (100) as claimed in claim 1, wherein the handle (10) has a tapered portion (14) arranged at the lower end (102) of the handle (10), wherein the camera channel (15) extends from the tapered portion (14) and includes a camera housing (16) arranged at a distal end to capture the visuals of the path of the endotracheal tube, larynx, trachea or airway.

8. The video laryngoscope (100) as claimed in claim 1, wherein the handle (10) has a receiving port (12) adapted to receive a display unit (30) therein, the receiving port (12) is configured to attach the display unit (30) to the handle (10) using an attachment assembly (25), the attachment assembly (25) is arranged within the receiving port (12) and is adapted to detachably receive the display unit (30) therein.

9. A handle (10) for a video laryngoscope (100), comprising:
a body (11) having an upper end (101) and a lower end (102), the upper end (101) is adapted to receive a display unit (30) through a receiving port (12) and connect the blade (20) to the handle (10), wherein the lower end (102) is configured to connect a camera channel (15) to the handle (10);
a controller (19) arranged within the body (11), connectable to the display unit (30) and the camera channel (15),
wherein, the controller (19) is adapted to connect with the display unit (30) through terminals exposed within the receiving port (12), establishing a connection between the display unit (30) and the camera channel (15) to send visuals of the path of the endotracheal tube, larynx, trachea, or airway to the display unit (30).

10. The handle (10) as claimed in claim 9, wherein the camera channel (15) has an image sensor (161) arranged at a distal end to capture the visuals of the trachea of the patient and communicate the visuals with the display unit (30) through the camera channel (15) and the controller (19) of the handle (10) when the display unit (30) is attached to the handle (10).

11. The handle (10) as claimed in claim 9, wherein the terminals include a first terminal (122) arranged within the receiving port (12) and a second terminal (28) arranged on the display unit (30), the first terminal (122) and the second terminal (28) are adapted to establish an electrical connection between the image sensor (161) and the display unit (30) to communicate and display the visual of the trachea of the patient on the display unit (30).

12. The handle (10) as claimed in claim 9, wherein the upper end (101) of the handle (10) has an attachment mechanism (21) arranged within a receiving port (12) to attach the blade (20) to the handle (10), the attachment mechanism (21) includes at least two ball projections (211a, 211b) extending radially away from a center of the handle (10), engageable with the blade (20) to securely hold the blade (20) in an attached position.

13. A blade (20) for a video laryngoscope (100), the video laryngoscope (100) having a handle (10) adapted to attach the blade (20) thereon, the blade (20) comprising:
an attaching portion (251);
a gripping portion (252) adjacent to the attaching portion (251); and
a tongue portion (253) extending from the gripping portion (252), adapted to insert into the trachea of the patient during the intubation process,
wherein, the blade (20) is configured to slide over the handle (10) and a camera channel (15), enclosing the handle (10) within a cavity formed at the attaching portion (251) and the gripping portion (252), wherein the attaching portion (251) is adapted to engage with an upper end (101) of the handle (10) to securely hold the blade (20) thereon and the gripping portion (252) provides a grip for the clinician during the intubation process.

14. The blade (20) as claimed in claim 13, wherein the blade (20) has at least two openings (223a, 223b) engageable with the handle (10) to hold the blade (20) around the handle (10), the handle (10) has an attachment mechanism (21) having at least two ball projections (211a, 211b) adapted to engage with the corresponding openings (223a, 223b) of the blade (20) to hold the blade (20) around the handle (10) in an attached position during the intubation process.

15. The blade (20) as claimed in claim 13, wherein the blade (20) has a first cavity (256) formed at the attaching portion (251) and the gripping portion (252) to accommodate the handle (10), and a second cavity (257) formed at the tongue portion (253) to accommodate a camera channel (15) within the blade (20).
